# EUROPÄISCHE PATENTSCHRIFT

(11) **EP 0 820 977 B1**
(45) Veröffentlichungstag und Bekanntmachung des Hinweises auf die Patenterteilung: **26.04.2000**
(21) Anmeldenummer: 97111019.2
(22) Anmeldetag: 02.07.1997
(51) Int. Cl.: C07C 45/67, C07C 49/293, C07D 307/28

(54) **Verfahren zur Herstellung von Cyclopropylalkylketonen und 4,5-Dihydroalkylfuranen**
Process for the preparation of cyclopropylalkylketones and 4,5-dihydroalkylfurans
Procédé pour la préparation de cyclopropylalkylcétones et de 4,5-dihydroalkylfuranes

(30) Priorität: 27.07.1996 DE 19630449
(43) Veröffentlichungstag der Anmeldung: 28.01.1998
(73) Patentinhaber: Degussa-Hüls Aktiengesellschaft, 60287 Frankfurt am Main (DE)
(72) Erfinder: Kaufhold, Manfred, Dr., 45770 Marl (DE); Köhler, Günther, Dr., 45770 Marl (DE); Feld, Marcel, Dr., 51147 Köln (DE)

(56) Entgegenhaltungen:
- EP-A- 0 552 586
- EP-A- 0 560 109
- EP-A- 0 725 066
- TAKEI S ET AL: "Novel synthesis of cyclopropyl ketones by decarboxylative ring contractions of.alpha.-acyl-.gamma.-butyrolactones catalyzed by halide ions in dipolar aprotic solvents" TETRAHEDRON LETT. (TELEAY);75; (49); PP.4389-92, TAKEDA CHEM. IND. LTD.;CENT. RES. DIV.; OSAKA; JAPAN, XP002003311
- ASAOKA M ET AL: "Alkylation reaction accompanied by dealkoxycarbonylation of.beta.-keto esters, geminal diesters, and.alpha.-cyano ester in hexamethylphosphoric triamide (HMPA)" CHEM. LETT. (CMLTAG);75; (11); PP.1149-52, TOKYO INST. TECHNOL.;LAB. ORG. CHEM.; TOKYO; JAPAN, XP002003310
- CHEMICAL ABSTRACTS, vol. 088, no. 21, 22.Mai 1978 Columbus, Ohio, US; abstract no. 152074, NEFEDOV O M ET AL: "Preparation of acetopropyl alcohol esters and their conversion to methyl cyclopropyl ketone" XP002045107 & IZV. AKAD. NAUK SSSR, SER. KHIM. (IASKA6,00023353);78; (1); PP.234-7, INST. ORG. KHIM. IM. ZELINSKOGO;MOSCOW; USSR,

## Beschreibung

Die Erfindung betrifft ein neues Verfahren zur gleichzeitigen Herstellung von Cyclopropylalkylketonen der Formel I und 4,5-Dihydroalkylfuranen der Formel II aus 3-Acyltetrahydrofuran-2-onen der Formel III nach dem Schema:

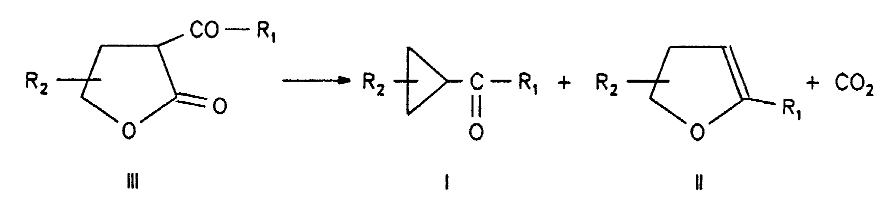

In diesem Reaktionsschema steht R₁ für Alkyl mit 1 bis 4 C-Atomen, Cyclohexyl oder Phenyl und R₂ für Wasserstoff, Alkyl mit 1 bis 4 C-Atomen oder Phenyl. Die Reaktion erfolgt dabei mit Hilfe eines Metallsalzes in einem hochsiedenden Lösemittel bei 160 bis 220 °C

Verbindungen der Formeln I und II sind bekannt. Sie dienen als wichtige Ausgangsverbindungen zur Herstellung von Pflanzenschutzmitteln und Pharmaprodukten.

Die Herstellung von Cyclopropylalkylketonen aus 3-Acyltetrahydrofuran-2-onen durch Decarboxylierungsreaktionen ist literaturbekannt. So wird für die Reaktion nach Takei, Tetrahedron Letters No. 49, 4389-92 (1975), immer ein 10%iger molarer Überschuß an Alkalihalogenid, bezogen auf das Substrat, und außerdem ein polares Lösemittel, wie Dimethylsulfoxid oder Dimethylformamid, angewandt. Bei diesem Verfahren ist die Rückgewinnung der großen Alkalihalogenidmengen besonders aufwendig, da sowohl das Metallsalz als auch das Lösemittel sehr gut wasserlöslich sind.

Bei Asaoka, Chemistry Letters 11, 1149-52 (1975), wird α-Acetyl-γ-butyrolacton mit unterschüssigem NaJ in Hexamethylphosphorsäuretriamid (HMPA), einem weiteren sehr polaren Lösemittel, umgesetzt. Nach der Destillation des Cyclopropylmethylketons erfordert die Rückgewinnung des Metallsalzes aus dem polaren Lösemittel auch hier ein aufwendiges Aufarbeitungsverfahren.

Nach EP-A-0 610 819 werden nur mit HMPA gute Ausbeuten erhalten, wobei das Lösemittel teuer und nicht unbedenklich sei. Im übrigen ist eine Gasphasenreaktion an einem Katalysatorbett Gegenstand dieser Anmeldung. Die Gasphasenreaktion erfordert jedoch eine spezielle Apparatur. Sie ist darüber hinaus mit einem großen technischen Aufwand verbunden, da der Katalysator auf einem festen Träger zuerst hergestellt und später regeneriert oder entsorgt werden muß.

In EP-A-0 552 586 wird ein großer Überschuß eines Halogenids in einem polaren Lösemittel, meist N-Methylpyrrolidon, vorgelegt, worauf bei der Reaktionstemperatur Acetylbutyrolacton zugetropft und Cyclopropylmethylketon destilliert wird. Wegen der großen Menge an Metallsalz und aus Umweltschutzgründen ist bei diesem Verfahren eine Rückgewinnung des Metallsalzes unbedingt erforderlich. Die Rückgewinnung ist dabei auch hier sehr aufwendig.

Aufgabe der vorliegenden Erfindung war es daher, ein einfaches Verfahren bereitzustellen, das in üblichen Rührapparaturen durchführbar ist, bei dem der Katalysator auf einfache Weise zurückgewonnen werden kann und bei dem nur wenig hochsiedende Abfallprodukte entstehen.

Die Aufgabe wird erfindungsgemäß dadurch gelöst, daß man für die Reaktion als Lösemittel ein Polyethylenglykoldialkylether der Formel IV 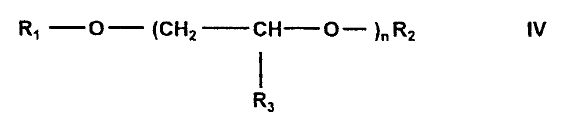 verwendet. Dabei stellen R₁ und R₂ Alkylreste mit 1 bis 4 C-Atomen dar, wobei R₁ und R₂ gleich oder verschieden sein können, R₃ steht für Wasserstoff oder eine Methylgruppe, und n ist eine Zahl von 20 bis 50. Statt IV alleine kann auch ein Gemisch aus IV und bis zu 50 %, bezogen auf die Gesamtlösemittelmenge, weiteren hochsiedenden, polaren und wasserlöslichen Lösemitteln eingesetzt werden. Außerdem ist die Reaktion in der Weise durchzuführen, daß man das Metallsalz und III im molaren Überschuß zum Metallsalz im Lösemittel oder Lösemittelgemisch vorlegt, auf 160 bis 220 °C erwärmt, dann weiteres III zugibt und dabei I und II abdestilliert.

Beispiele für Cyclopropylalkylketone I sind Cyclopropylmethylketon, Cyclopropylethylketon, Cyclopropylphenylketon, Cyclopropylcyclohexylketon und 1-Methyl-cyclopropylphenylketon.

Beispiele für 4,5-Dihydroalkylfurane II sind 4,5-Dihydro-2-methylfuran, 4,5-Dihydro-2,5-dimethylfuran, 4,5-Dihydro-2-phenylfuran, 4,5-Dihydro-2-cyclohexylfuran und 4,5-Dihydro-5-methyl-2-phenylfuran.

Geeignete Ausgangsverbindungen III sind beispielsweise α-Acetyl-γ-butyrolacton, 3-Acetyl-5-methyltetradydiofuran-2-on, α-Benzoyl-γ-butyrolacton, α-Acetyl-α-phenyl-γ-butyrolacton und α-Benzoyl-α-methyl-γ-butyrolacton.

Als Metallsalze kommen vor allem Alkali- und Erdalkalihalogenide in Betracht. Dabei werden Alkalihalogenide vorzugsweise eingesetzt. Beispiele dafür sind LiBr, LiJ, KCl, NaBr, KBr, NaJ und KJ, wobei NaJ und KJ ganz besonders bevorzugt werden.

In dem verwendeten Lösemittel werden Metallsalz und III vorzugsweise im molaren Verhältnis 1 : 2 bis 1 : 10 und im besonderen im molaren Verhältnis von 1 : 2,5 bis 1 : 5 vorgelegt.

Bei den Lösemitteln IV handelt es sich um hochsiedende, polare und wasserlösliche Verbindungen. Beispiele dafür sind Polyethylenglykoldimethylether, Polyethylenglykoldiethylether oder Polypropylenglykoldimethylether.

Wegen ihres hohen Siedepunktes ermöglichen die Lösemittel eine einfache Rückführung des Metallsalzes aus den als Nebenprodukt anfallenden Hochsiedern. Man kann das Metallsalz nämlich zusammen mit dem Lösemittel mit Wasser extrahieren und dann das Wasser des Extraktes abdestillieren. Das im Sumpf verbleibende Lösemittel IV löst dann das wasserfrei anfallende Metallsalz.

Die weiteren Lösemittel, die IV bis zu 50 % substituieren können, sind beispielsweise Dimethylformamid, Dimethylsulfoxid oder N-Methylpyrrolidon.

Wesentlich für das erfindungsgemäße Verfahren ist, daß III im molaren Überschuß zum Metallsalz vorgelegt wird. Das ergeben die experimentellen Daten. Bei diesem Überschuß können dann die Produkte I und II mit Hilfe der speziellen Lösemittel überraschend einfach und wirtschaftlich hergestellt werden.

Im Gegensatz zu der in EP-A-0 610 819 geäußerten Auffassung werden auch mit den weniger polaren Lösemitteln hohe Ausbeuten erzielt. Anders als im Stand der Technik entstehen hochsiedende Produkte nur in geringen Mengen. Statt dessen fällt II als weiteres leicht siedendes Produkt an. Die geringen Mengen an Hochsiedern haben den wirtschaftlichen Vorteil, daß der Sumpf seltener aufgearbeitet werden muß und daß daher die Standzeit bis zum Abbruch der Reaktion erhöht wird.

Es wird also weniger Salz eingesetzt, das seltener und dann in einfacher Weise zurückgewonnen wird. Gleichzeitig gewinnt man mit II ein weiteres wertvolles Zwischenprodukt.

Zur Durchführung des erfindungsgemäßen Verfahrens legt man das Lösemittel oder ein Lösemittelgemisch und III vor und gibt dann das Metallsalz hinzu. Die Konzentration des Metallsalzes beträgt meist 5 bis 20 %, bezogen auf das Gemisch aus Lösemittel und III. Im besonderen wird ein Molverhältnis von Metallsalz und III von 1 : 2 eingestellt. Man erwärmt auf 160 bis 220 °C, vorzugsweise auf 180 bis 200 °C und dosiert weiteres III zu. Das dann sich bildende I und II wird abdestilliert.

Die folgenden Beispiele sollen die Erfindung verdeutlichen.

### Vergleichsbeispiel A

In einer Glasapparatur aus einem Dreihalskolben mit Rührer, Thermometer, Destillationskolonne, Destillationsbrücke und Vorlage werden eingesetzt:

| | |
|---|---|
| 100 g | 1-(N-Octyl)caprolactam (NOC) |
| 40 g (0,31 Mol) | 3-Acetylbutyrolacton (ABL) |
| 15 g (0,1 Mol) | NaJ, getrocknet |

Die Produkte werden vermischt und auf 180 °C erhitzt. Dabei fällt kein Destillat an.

### Beispiel 1

Man benutzt die im Vergleichsbeispiel A beschriebene Apparatur und legt vor: 300 g Polyethylenglykoldimethylether 2000 (beidseitig methylgruppenverschlossenes Polyethylenglykol M = 2000), 30 g NaJ (0,2 mol) und 60 g 2-Acetyl-y-butyrolacton (0,47 mol). Dann wird auf 175 - 180 °C erhitzt.

Gleichzeitig wird mit der kontinuierlichen Dosierung von 2-Acetyl-γ-butyrolacton begonnen, wobei 60 g/h (0,47 mol) mittels Dosierpumpe gefördert werden. Entsprechend der Eduktmenge werden stündlich ca. 40 g Rohcyclopropylmethylketon und ca. 10 l CO₂ gebildet.

Nach Reindestillation dieses Rohgemisches werden, bezogen auf die stündlich dosierten 0,47 mol 2-Acetyl-γ-butyrolacton, 34,5 g Cyclopropylmethylketon (99 %ig) gewonnen, was einer Ausbeute von ca. 87 % (d. Th.) entspricht. Die Ausbeute an 4,5-Dihydro-2-methylfuran, das bei der Destillation als Vorlauf erhalten wird, beträgt 8,5%.

## Patentansprüche

1. Verfahren zur gleichzeitigen Herstellung von Cyclopropylalkylketonen der Formel I und Alkyl-4,5-dihydro-2-alkylfuranen der Formel II aus 3-Acyltetrahydrofuran-2-onen der Formel III 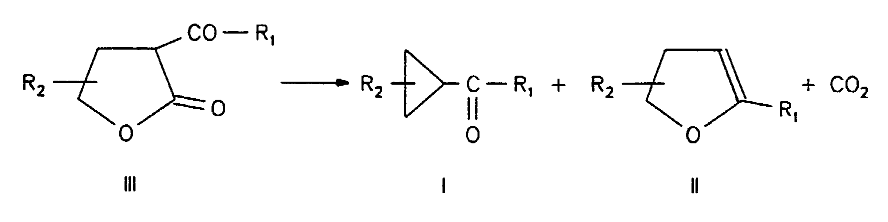 wobei
R₁ = Alkyl mit 1 bis 4 C-Atomen, Cyclohexyl oder Phenyl und
R₂ = H, Alkyl mit 1 bis 4 C-Atomen oder Phenyl ist,
durch Umsetzung mit einem Metallsalz in einem hochsiedenen Lösemittel bei 160 bis 220 ° C,
dadurch gekennzeichnet,
daß man als Lösemittel ein Polyethylenglykoldialkylether der Formel IV 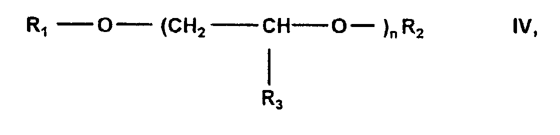 wobei
R₁, R₂ = Alkyl mit 1 bis 4 C-Atomen,
R₃ = H oder Methyl und
n = 20 bis 50 ist,
oder ein Gemisch aus IV und bis zu 50 %, bezogen auf die gesamte Lösemittelmenge, weiteren hochsiedenden, polaren und wasserlöslichen Lösemitteln verwendet und für die Reaktion das Metallsalz und III im molaren Überschuß zum Metallsalz im Lösemittel oder im Lösemittelgemisch vorlegt, auf 160 bis 220 ° C erwärmt, dann weiteres III zugibt und dabei I und II abdestilliert.

2. Verfahren nach Anspruch 1,
dadurch gekennzeichnet,
daß als Metallsalz ein Alkalihalogenid eingesetzt wird.

3. Verfahren nach Anspruch 2,
dadurch gekennzeichnet,
daß NaJ oder KJ verwendet wird.

4. Verfahren nach Anspruch 1,
dadurch gekennzeichnet,
daß Metallsalz und III im molaren Verhältnis von 1 : 2 bis 1 : 10, vorzugsweise von 1 : 2,5 bis 1 : 5 vorgelegt werden.

5. Verfahren nach Anspruch 1,
dadurch gekennzeichnet,
daß das Metallsalz nach der Destillation von I und II durch Wasserwäsche zurückgewonnen wird.

## Claims

1. A process for simultaneously preparing cyclopropyl alkyl ketones of the formula I and alkyl-4,5-dihydro-2-alkylfurans of the formula II from 3-acyltetrahydrofuran-2-ones of the formula III 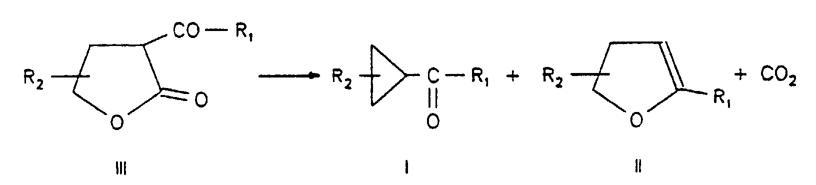 where
R₁ = alkyl having 1 to 4 carbon atoms, cyclohexyl or phenyl and
R₂ = H, alkyl having 1 to 4 carbon atoms or phenyl,
by reaction with a metal salt in a high-boiling solvent at 160 to 220°C,
characterized in that the solvent used is a poly(ethylene glycol) dialkyl ether of the formula IV 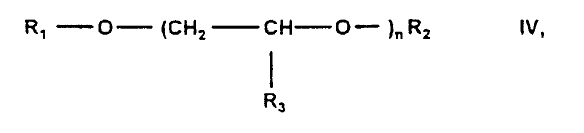 where
R₁, R₂ = alkyl having 1 to 4 carbon atoms,
R₃ = H or methyl and
n = 20 to 50,
or a mixture of IV and up to 50%, based on the total amount of solvent, of further high-boiling, polar, water-soluble solvents, and, for the reaction, the metal salt and III in a molar excess to the metal salt are introduced in the solvent or in the solvent mixture, the mixture is heated to 160 to 220°C, then further III is added and I and II are thereby distilled off.

2. A process according to claim 1, characterized in that the metal salt used is an alkali metal halide.

3. A process according to claim 2, characterized in that NaI or KI is used.

4. A process according to claim 1, characterized in that metal salt and III are introduced in a molar ratio of 1 : 2 to 1 : 10, preferably 1 : 2.5 to 1 : 5.

5. A process according to claim 1, characterized in that the metal salt is recovered after the distillation of I and II by washing with water.

## Revendications

1. Procédé de préparation simultanée de cyclopropylalkylcétones de formule I et d'alkyl-4,5-dihydro-2-alkylfuranes de formule II à partir de 3-acyltétrahydrofurane-2-ones, de formule III, 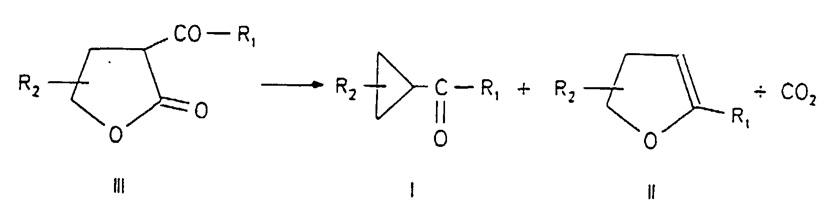 dans laquelle,
R₁ est un alkyle ayant de 1 à 4 atomes de carbone, un cyclohexyle ou un phényle et
R₂ = H, alkyle, ayant de 1 à 4 atomes de carbone ou un phényle,
par réaction avec un sel métallique dans un agent dissolvant à haut point d'ébullition de 160 à 220°C,
caractérisé en ce que
• comme agent dissolvant on utilise un éther de dialkyle de polyéthylèneglycol de formule IV 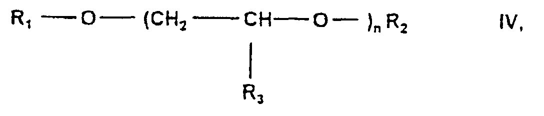 dans laquelle
R₁, R₂ sont un alkyle ayant de 1 à 4 atomes de carbone,
R₃ est H ou méthyle et,
n est 20 à 50,
ou un mélange de IV et jusqu'à 50 % rapporté à la quantité totale d'agent dissolvant, d'autres agents dissolvants à point d'ébullition élevé, polaires et solubles dans l'eau,
• on introduit pour la réaction, le sel métallique et III en excès molaire par rapport au sel métallique dans l'agent dissolvant ou dans le mélange d'agents dissolvants,
• on chauffe de 160 à 220°C puis on ajoute davantage de III, et de cette façon
• on sépare par distillation, I et II.

2. Procédé selon la revendication 1,
caractérisé en ce qu'
on met en oeuvre comme sel métallique, un halogénure de métal alcalin.

3. Procédé selon la revendication 2,
caractérisé en ce qu'
on utilise INa ou IK.

4. Procédé selon la revendication 1,
caractérisé en ce qu'
on introduit le sel métallique et III en rapport molaire allant de 1:2 à 1:10, de préférence de 1:2,5 à 1:5.

5. Procédé selon la revendication 1,
caractérisé en ce qu'
on récupère le sel métallique par lavages à l'eau, après la distillation de I et de II.
